# EUROPEAN PATENT SPECIFICATION

(11) **EP 2 833 789 B1**
(45) Date of publication and mention of the grant of the patent: **27.02.2019**
(21) Application number: 13724330.9
(22) Date of filing: 21.03.2013
(51) Int. Cl.: A61B 5/09, G01F 1/12, A61M 16/00

(54) **SPIROMETER**
SPIROMETER
SPIROMÈTRE

(30) Priority: 03.04.2012 GB 201205952
(43) Date of publication of application: 11.02.2015
(73) Proprietor: Medchip Solutions Limited, Bromley, Kent BR1 3LX (GB)
(72) Inventor: LAWSON, Christopher, Bromley Kent BR1 3LX (GB); FOXWELL, Ian, Hoo St Werburgh ME3 9HU (GB)
(74) Representative: Jaeger, Michael David
(86) International application number: PCT/GB2013/050743
(87) International publication number: WO 2013/150267

(56) References cited:
- US-A1- 2006 060 199
- US-A1- 2012 029 376

## Description

### FIELD OF THE INVENTION

This invention relates to a spirometer and in particular to a turbine spirometer.

A spirometer is a medical diagnostic device that measures the flow and volume of a subject's forced exhaled and/or inhaled breath. Spirometers are used to diagnose common respiratory diseases. Examples of such diseases are asthma and Chronic Obstructive Pulmonary Disease (COPD).

### BACKGROUND OF THE INVENTION

Turbine spirometers have been available since the mid-1980s. An example of a turbine spirometer is described in UK patent application published as GB-A-2224567, in the name of Micro Medical Limited.

Turbine-based spirometers include a mouth piece through which a subject blows (or inhales). Beyond the mouth piece is a fixed swirl plate which causes the exhaled air to swirl, twisting the air into a vortex. The angular velocity of the vortex is proportional to the flow rate of air passing through the mouth piece. The spirometer further includes a vane beyond the swirl plate. The vane may be a flat rectangular piece of plastic film mounted on an axle. The swirled air applies a torque to the vane which rotates at the same angular velocity as the vortex. The angular velocity of the vane is thus proportional to the flow of air within the tube. A light source, such as an infra red transmitter, transmits a continuous infra red beam. The infra red beam is interrupted by the vane as it rotates, resulting in infra red pulses. An infra red receiver receives the infra red pulses. Electronic circuitry processes the received infra red pulses to determine the volume and flow rate of the air exhaled by the subject.

In order to work effectively spirometers should be capable of operating over a large range of flow rates. This is particularly important when diagnosing certain conditions such as COPD, where the subject is only capable of exhaling at a low flow rate for a substantial portion of the forced expiration. Standards for spirometry testing are specified in Standardisation of Spirometry (published in volume 26, number 2 of the European Respiratory Journal of 2005). The standard specifies that spirometers should be capable of responding to flows as low as 0.025 L/s. This very low flow rate requires a high sensitivity from the spirometer for a protracted period of time whilst the subject exhales.

The vane of conventional spirometers is disposed so that its axis is coaxial with the mouth piece, i.e., horizontal in use. This results in a short and straight air flow path. The axial shaft of the vane is conventionally mounted by means of jewel bearings, which are ideally suited for low speed and low load applications due to their low coefficients of friction. Nevertheless, the response of a turbine-based spirometer at low flow rate is limited by friction in these supporting bearings. Further, any imbalance in the vane assembly limits the turbine's ability to respond to very low flow rates. Since the torque on the vane is proportional to the flow, at low flow rates there is a correspondingly low torque available to turn the vane assembly. This means that if there is imbalance in the vane then the low torque may be insufficient to raise a heavier side of an unbalanced vane and the rotation will cease whilst the heavier side is below the pivot when the axis of the turbine is horizontal.
One way of improving the rotation of a vane is to support the vane using V-jewel bearing, which exhibit very low friction. However when these low friction bearings are used in conventional turbine spirometers, the pivot lies against the inside of the conical V and rolls up and down the surface of the bearing radius as the vane rotates. This means that some of the available torque is lost as the pivot rolls up the inside cone of the V-jewel bearing rather than in the nadir of the V.

US-A-2006/0060199 describes a self-inflating resuscitation system.

US-A-2012/0029376 discloses the preamble of claim 1.

The present invention aims to provide an improved turbine-based spirometer in which the vane can freely rotate at low flow volumes.

According to a first aspect of the invention there is provided a turbine spirometer comprising: a breathing tube having an opening into which a subject is to breathe, the breathing tube having a first axis at the opening in the direction of air flow, a swirl plate for swirling the breath into a vortex, and a two-dimensional vane mounted on a shaft, the vane to be driven by the swirled breath to rotate about a second axis, wherein the first and second axes are inclined to one another at angle of 90° ± 50°.

The angle of inclination may be 100° ± 40°, 100° ± 20° or 100° ± 10° when the spirometer is adapted to be used with the vane below the breathing tube.
The angle of inclination may be 80° ± 40°, 80° ± 20° or 80° ± 10° when the spirometer is adapted to be used with the vane above the breathing tube.
The first and second axes may be co-planar.

According to a second aspect of the disclosure there is provided a turbine spirometer having an air flow cavity having a first opening adapted for a subject to breathe through, a swirl plate and a two-dimensional vane rotatably mounted within the cavity, the cavity being shaped such that in use a direction of air flow in the cavity is diverted through an angle of 90° ± 50° between the first opening and the vane.

The spirometer may further comprise a mouth piece removably attached to the breathing tube or cavity. The mouth piece forms part of the breathing tube or cavity wall.

According to a third aspect of the disclosure there is provided a turbine spirometer comprising a vane which, in use, rotates about a substantially vertical axis when a subject is sitting or standing.
There is provided a swan neck-shaped or elbow-shaped turbine spirometer mouth piece. There is provided a spirometer mouth piece having a first opening adapted for a subject to blow through and a second opening adapted for connection to a spirometer, wherein the direction of air flow at the first opening is inclined relative to the direction of air flow at the second opening. The two directions may be co-planar.
The invention will be further described by way of example with reference to the accompanying drawings in which:
Figure 1 is a perspective partly cut-away view of a spirometer forming a first embodiment of the invention; and
Figure 2 is a perspective partly cut-away view of a spirometer forming a second embodiment of the invention.

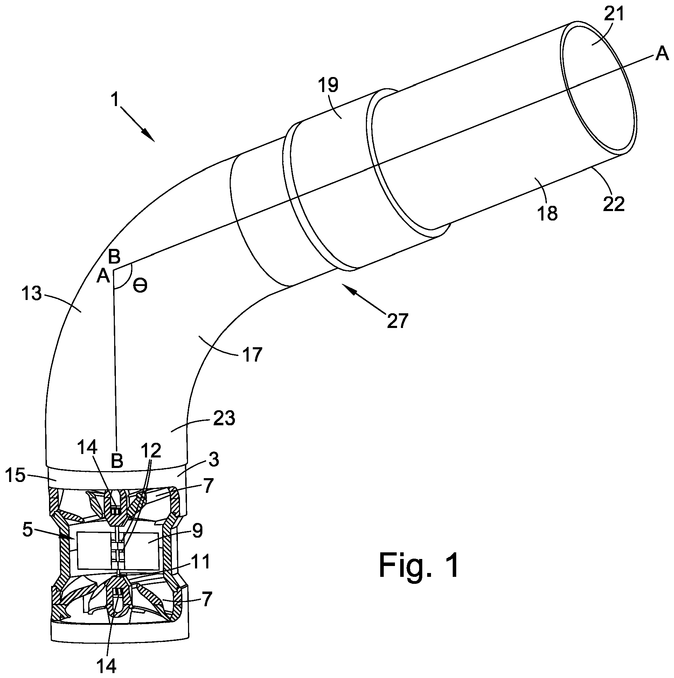

In Figure 1, a spirometer 1 comprises a turbine assembly housing 3 in which is mounted a vane assembly 5 and two swirl plates 7. The turbine assembly housing 3 is partially cut-away to show part of the vane assembly 5 and the swirl plates 7. The vane assembly 5 comprises a vane 9 mounted on a shaft 11. The vane 9 is made of a rectangular flat/two-dimensional piece of thin plastic film and typically weighs around 0.07g. The vane 9 has slits 12 in its centre through which the shaft 11 is threaded. The ends of the shaft 11 rest in respective V-jewel bearings 14 in each of the swirl plates. The vane 9 and shaft 11 are mounted in the swirl plates 7 so as to rotate in the turbine assembly housing 3 about the shaft 11 as air impels the vane. The moment of inertia of the vane assembly 5 is around 0.015g.mm².

The spirometer 1 further comprises a bent tube 13 which is attached to the upper end 15 of the turbine assembly housing 3. The bent tube 13 has a generally circular cross section which is substantially constant along its length and includes a bend 17 in its middle portion such that it has the overall shape of a swan neck or elbow. At the upper end 19 of the bent tube a detachable mouth piece 18 is attached by means of a compression/friction fit. The mouth piece 18 has an opening 21 through which a subject breathes when measuring the flow and volume of subject's exhaled (and/or inhaled) breath. The axis of this portion 22 of the mouth piece 18 is shown in Figure 1 as line A-A. The lower portion 23 of the bent tube 13 is connected to the turbine assembly housing 3 and the axis of this portion 23 of the bent tube 13 is indicated by line B-B, which is coaxial with the shaft 11. These two lines, A-A and B-B lie in the same plane and intersect at an angle θ. In this embodiment θ is around 100°. The mouth piece 18 is removably attached to the bent tube 13 so that a new mouth piece 18 can be used for each subject, for hygiene reasons.

The spirometer 1 also includes sensors (not shown) for detecting the rotation of the vane, as is known in the art. The sensors may utilise infra red beams, as discussed in the introduction to this application and as will be apparent to those skilled in the art.

In use, when the flow and volume of a subject's exhaled (or inhaled) breath is to be measured, a new mouth piece 18 is attached to the upper end 19 of the bent tube 13. The subject seals their lips around the opening 21 of the mouth piece 18, exhales through the mouth piece 18 so that their breath passes along the upper portion 19 of the bent tube 13 in the direction of line A-A and around the bend 17 in the bent tube 13 so that the direction of the breath is diverted to travel in the direction of line B-B as it enters the turbine assembly housing 3. As the breath enters the turbine assembly housing 3 it passes through the upper swirl plate 7 which swirls the air into a vortex. The vortex applies a torque to the vane 9 which then rotates, together with the shaft 11, at the same angular velocity as the vortex. The rotation of the vane 9 and shaft 11 is detected by the detecting means (not shown) of the spirometer 1.

In certain diagnostic tests, the subject is required to inhale through the spirometer 1. In these cases, as the subject inhales, air enters the spirometer 1 through the lower swirl plate 7. The vortex produced by the lower swirl plate 7 causes the vane 9 to rotate, thereby rotating the vane assembly 5. The rotation of the vane assembly 5 is detected by the detecting means as described above.

An advantage of the present invention is that in use, whilst the subject holds and breathes through the spirometer 1, the vane 9 rotates about a vertical axis, corresponding to the shaft 11. Since the vane 9 rotates about a vertical axis, any imbalance in either side of the vane does not affect the free rotation of the vane 9. Furthermore, since the shaft 11 on which the vane 9 is mounted is vertical, its lower end rests at the nadir of the V-jewel bearing 14 in the lower swirl plate 7.

By orienting the vane 9 in use horizontally, i.e., so that it rotates about a vertical axis, as the air flow causes the vane 9 to rotate, the vane 9 is able to maintain its rotation at low speeds and under a low flow rate.

It will be readily apparent to the skilled person that by introducing a bend 17 in the bent tube 13 leading to the vane assembly 5, when a standing or seated subject breathes into the spirometer 1, the shaft 11 of the vane assembly 5 is oriented vertically in use so that the air flow which comes from the mouth of the subject is diverted from a generally horizontal direction to pass through the vane assembly 5 in a generally vertical direction.

The angle θ is chosen to be around 100° rather than around 90° since it is normal for the subject to lean forward slightly when breathing into the spirometer 1. By choosing an angle of around 100° this will mean that the shaft 11 of the turbine assembly 3 will be near vertical in use.

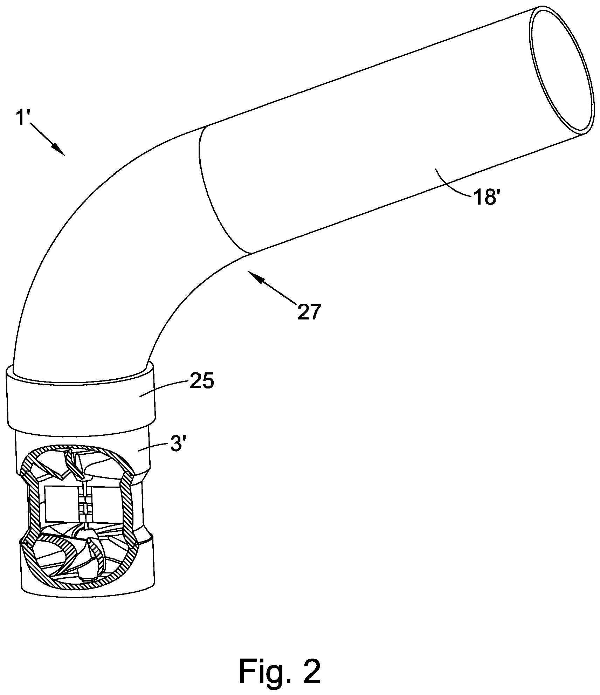

In the embodiment of Figure 1, the change of direction of the air flow within the spirometer is achieved using a bent tube 13. The bent tube 13 and mouth piece 18 are together considered as a breathing tube 27 of the spirometer 1', i.e., the air passage from the opening 21 of the mouth piece 18 to where the functional part of the vane assembly 5 begins. It will be readily apparent to the skilled person that the bend 17 needs to be somewhere along the path from the opening 21 of the mouth piece 18 to the vane assembly 5. A spirometer according to a second embodiment of the invention in line with this concept is shown in Figure 2.

Figure 2 shows a spirometer 1' which is similar to the spirometer 1 of Figure 1. The spirometer 1' of Figure 2 differs from the spirometer 1 of Figure 1 in that it has a removable mouth piece 18' which is longer than the mouth piece 18 of Figure 1 and which is bent to connect to a collar 25 at the upper end of the turbine assembly housing 3'. The mouth piece 18' is considered as the breathing tube 27' of the spirometer 1', i.e., the air passage from the opening 21 of the mouth piece 13' to where the functional part of the vane assembly 5 begins.

Consequently, although the mouth piece 18' and turbine assembly housing 3' each have a different shape to their counterparts in Figure 1, the overall shape of the spirometer 1' of Figure 2 is very similar to the overall shape of the spirometer 1 of Figure 1. The skilled person will understand that other configurations of mouth piece, tubing and turbine assembly housing in combination can achieve the function of a breathing tube which diverts breathed air from and to a subject's mouth to and from a vertically mounted turbine assembly.

Various modifications will be apparent to those in the art and it is desired to include all such modifications as fall within the scope of the accompanying claims.

For example, a spirometer embodying the invention may not include a removable mouth piece and instead the inlet tube of the turbine assembly housing may extend so that the spirometer has the overall shape of the spirometers shown in Figures 1 and 2. In this case, the extended inlet tube can be said to be the breathing tube of the spirometer.

Alternatively, the inlet tube of the spirometer may have a shape as described immediately above and a removable mouth piece may be used at the opening of the inlet tube for reasons of hygiene.

In the embodiments described above, the spirometer is used such that the turbine assembly is held below the mouth piece. In other embodiments the spirometer may be adapted so that the turbine assembly is held above the mouth piece, i.e., in front of the nose and eyes. In this case since the subject will be leaning slightly forward when breathing into the spirometer, the angle between the direction of flow of air into the spirometer mouth piece and the axis of vertical rotation of the vane will be around 80°.

In a preferred embodiment the axis of rotation of the vane is vertical in use. In other embodiments the angle of inclination of the axis of rotation in use may be up to 10°, up to 20° or up to 40° to the vertical. It is understood that whilst the angle of inclination of the axis of rotation is in this range the disadvantages of the having the axis of rotation horizontal are at least partially overcome.

If the above ranges are applied to the first two embodiments of the invention where the turbine assembly housing is held below the mouth piece then this results in spirometers where the angle of inclination between the axis of rotation of the vane and the direction of air flow into the mouth piece is 100° ± 40°, 100° ± 20° and preferably 100° ± 10°.

If the above ranges are applied to an embodiment of the invention where the turbine assembly housing is held above the mouth piece then this results in spirometers where the angle of inclination between the axis of rotation of the vane and the direction of air flow into the mouth piece is 80° ± 40°, 80° ± 20° and preferably 80° ± 10°.

The skilled person will understand that when references are made to the direction of flow of air through the spirometer, the references should be understood to mean that general direction of air flow and not the direction of particular parts of the air flow. For example, as air is blown from the mouth into the mouth piece it may be the case that the air flow is non-laminar due to the shape of the tongue and teeth. Similarly, when the air passes through the swirl plate the direction of flow of individual components of the air will change from being parallel to the overall direction of flow of the air to result in a vortex, where each part of the air is moving in a different direction. Nevertheless, since the general direction of the flow of the vortex is in a particular overall direction, it is the latter which is the intention of this phrase.

In the first two embodiments, the breathing tube (mouth piece of Figure 1) is described as being swan neck-shaped or elbow-shaped. This is understood to mean that it has two substantially straight portions which are joined by a bent central portion. The skilled person will understand that the expressions 'swan neck-shaped' and "elbow-shaped" should not be limited to the specific example shown in the Figures and should be understood to include any shape where the axes at the ends of the tube are inclined to one another as described above. For example, the tube may be curved with a constant radius along its length, i.e., without straight portion at one or both of its ends. Alternatively the breathing tube may be curved such that it goes back on itself, for example in the general shape of a question mark, as long as the axes at the ends of the tube are inclined to one another as described above.

A swan neck-shaped or elbow-shaped mouth piece may be used with a conventional turbine spirometer to convert it into a spirometer in which the angle of air entry is diverted in the mouth piece such that it impacts the vane of the spirometer at an angle inclined to the direction of air entry as described above.

The spirometer shown in Figures 1 and 2 has two swirl plates at each end of the turbine assembly housing, which means that it can be used to test both inhalation and exhalation. In other embodiments the spirometer may include a single swirl plate so that the spirometer is used for only one of these two functions.

## Claims

1. A turbine spirometer (1, 1') comprising:
a breathing tube (18, 18') having an opening (21) into which a subject is to breathe, the breathing tube (18, 18') having a first axis (A-A) at the opening (21) in the direction of air flow,
a swirl plate (7) for swirling the breath into a vortex, and
a two-dimensional vane (9) mounted on a shaft (11), the vane (9) to be driven by the swirled breath to rotate about a second axis (B-B),
**characterized in that** the first (A-A) and second (B-B) axes are inclined to one another at angle (θ) of 90° ± 50°.

2. A turbine spirometer (1, 1') as claimed in claim 1, wherein the angle of inclination (θ) is 100° ± 40°.

3. A turbine spirometer (1, 1') as claimed in claim 2, wherein the angle of inclination (θ) is 100° ± 20°.

4. A turbine spirometer as claimed in claim 3, wherein the angle of inclination (θ) is 100° ± 10°.

5. A turbine spirometer as claimed in any one of the preceding claims, wherein in use the vane (9) is below the breathing tube (18).

6. A turbine spirometer as claimed in claim 1, wherein the angle of inclination (θ) is 80° ± 40°.

7. A turbine spirometer as claimed in claim 6, wherein the angle of inclination is 80° ± 20°.

8. A turbine spirometer as claimed in claim 7, wherein the angle of inclination is 80° ± 10°.

9. A turbine spirometer as claimed in claim 6, 7 or 8, wherein in use the vane (9) is above the breathing tube (18).

10. A turbine spirometer as claimed in any one of the preceding claims, wherein the first (A-A) and second (B-B) axes are co-planar.

11. A turbine spirometer as claimed in any one of the preceding claims, further comprising a mouth piece (18) removably attached to the breathing tube (18) or cavity.

12. A turbine spirometer as claimed in claim 11, wherein the mouth piece (18) forms part of the breathing tube (18) or cavity wall.

## Patentansprüche

1. Ein Turbinenspirometer (1, 1'), umfassend:
ein Atemrohr (18, 18') mit einer Öffnung (21), in die ein Subjekt zu atmen hat, wobei das Atemrohr (18, 18') eine erste Achse (A-A) an der Öffnung (21) in der Luftströmungsrichtung aufweist,
eine Verwirbelungsplatte (7) zum Verwirbeln des Atems in einen Vortex und
eine zweidimensionale Schaufel (9), die auf einer Achse (11) befestigt ist, wobei die Schaufel (9) mittels des verwirbelten Atems anzutreiben ist, um sich um eine zweite Achse (B-B) zu drehen,
**dadurch gekennzeichnet, dass**
die erste (A-A) und die zweite (B-B) Achse in einem Winkel (θ) von 90° ± 50° zueinander geneigt sind.

2. Ein Turbinenspirometer (1, 1') nach Anspruch 1, wobei der Neigungswinkel (θ) 100° ± 40° beträgt.

3. Ein Turbinenspirometer (1, 1') nach Anspruch 2, wobei der Neigungswinkel (θ) 100° ± 20° beträgt.

4. Ein Turbinenspirometer nach Anspruch 3, wobei der Neigungswinkel (θ) 100° ± 10° beträgt.

5. Ein Turbinenspirometer nach einem der vorangehenden Ansprüche, wobei bei einer Verwendung die Schaufel (9) unterhalb des Atemrohrs (18) ist.

6. Ein Turbinenspirometer nach Anspruch 1, wobei der Neigungswinkel (θ) 80° ± 40° beträgt.

7. Ein Turbinenspirometer nach Anspruch 6, wobei der Neigungswinkel 80° ± 20° beträgt.

8. Ein Turbinenspirometer nach Anspruch 7, wobei der Neigungswinkel 80° ± 10° beträgt.

9. Ein Turbinenspirometer nach Anspruch 6, 7 oder 8, wobei bei einer Verwendung die Schaufel (9) oberhalb des Atemrohrs (18) ist.

10. Ein Turbinenspirometer nach einem der vorhergehenden Ansprüche, wobei die erste (A-A) und die zweite (B-B) Achse koplanar sind.

11. Ein Turbinenspirometer nach einem der vorhergehenden Ansprüche, weiter umfassend ein Mundstück (18), das an dem Atemrohr (18) oder dem Hohlraum entfernbar befestigt ist.

12. Ein Turbinenspirometer nach Anspruch 11, wobei das Mundstück (18) einen Teil des Atemrohrs (18) oder der Hohlraumwandung bildet.

## Revendications

1. Spiromètre à turbine (1, 1') comprenant :
un tube respirateur (18, 18') ayant une ouverture (21) dans laquelle un sujet doit respirer, le tube respirateur (18, 18') ayant un premier axe (A-A) à l'ouverture (21) dans la direction de l'écoulement de l'air,
une plaque tourbillon (7) pour faire tourbillonner la respiration et lui communiquer un vortex, et
une aube bidimensionnelle (9) montée sur un arbre (11), l'aube (9) étant mue par la respiration tourbillonnante pour tourner autour d'un second axe (B-B),
**caractérisé en ce que**
le premier (A-A) et le second (B-B) axes sont inclinés l'un par rapport à l'autre avec un angle (Θ) de 90° ± 50°.

2. Spiromètre à turbine (1,1') selon la revendication 1, dans lequel l'angle d'inclinaison (Θ) est de 100° ± 40°.

3. Spiromètre à turbine (1,1') selon la revendication 2, dans lequel l'angle d'inclinaison (Θ) est de 100° ± 20°.

4. Spiromètre à turbine selon la revendication 3, dans lequel l'angle d'inclinaison (Θ) est de 100° ± 10°.

5. Spiromètre à turbine selon l'une quelconque des revendications précédentes, dans lequel en utilisation, l'aube (9) se trouve sous le tube respirateur (18).

6. Spiromètre à turbine selon la revendication 1, dans lequel l'angle d'inclinaison (Θ) est de 80° ± 40°.

7. Spiromètre à turbine selon la revendication 6, dans lequel l'angle d'inclinaison est de 80° ± 20°.

8. Spiromètre à turbine selon la revendication 7, dans lequel l'angle d'inclinaison est de 80° ± 10°.

9. Spiromètre à turbine selon la revendication 6, 7 ou 8, dans lequel en utilisation, l'aube (9) est au-dessus du tube respirateur (18).

10. Spiromètre à turbine selon l'une quelconque des revendications précédentes, dans lequel le premier (A-A) et le second (B-B) axes sont coplanaires.

11. Spiromètre à turbine selon l'une quelconque des revendications précédentes, comprenant en outre une pièce d'embouchure (18) fixée de façon amovible au tube respirateur (18) ou à la cavité.

12. Spiromètre à turbine selon la revendication 11, dans lequel la pièce d'embouchure (18) fait partie du tube respirateur (18) ou de la paroi de la cavité.
